# EUROPÄISCHE PATENTSCHRIFT

(11) **EP 1 490 372 B1**
(45) Veröffentlichungstag und Bekanntmachung des Hinweises auf die Patenterteilung: **16.01.2008**
(21) Anmeldenummer: 03744812.3
(22) Anmeldetag: 19.03.2003
(51) Int. Cl.: C07D 487/04, A01N 43/90, C07C 255/57, C07C 323/62

(54) **FUNGIZIDE TRIAZOLOPYRIMIDINE, VERFAHREN ZU IHRER HERSTELLUNG UND IHRE VERWENDUNG ZUR BEKÄMPFUNG VON SCHADPILZEN SOWIE SIE ENTHALTENDE MITTEL**
FUNGICIDAL TRIAZOLOPYRIMIDINES, METHODS FOR PRODUCING THE SAME, USE THEREOF FOR COMBATING HARMFUL FUNGI AND AGENTS CONTAINING SAID SUBSTANCES
TRIAZOLOPYRIMIDINES FONGICIDES, LEUR PROCEDE DE PRODUCTION ET LEUR UTILISATION POUR LUTTER CONTRE DES CHAMPIGNONS NUISIBLES, ET AGENTS LES CONTENANT

(30) Priorität: 21.03.2002 DE 10212739; 10.04.2002 DE 10215814; 11.12.2002 DE 10258050
(43) Veröffentlichungstag der Anmeldung: 29.12.2004
(73) Patentinhaber: BASF Aktiengesellschaft, 67056 Ludwigshafen (DE)
(72) Erfinder: TORMO I BLASCO, Jordi, 69514 Laudenbach (DE); BLETTNER, Carsten, 68165 Mannheim (DE); MÜLLER, Bernd, 67227 Frankenthal (DE); GEWEHR, Markus, 68165 Mannheim (DE); GRAMMENOS, Wassilios, 67071 Ludwigshafen (DE); GROTE, Thomas, 67157 Wachenheim (DE); GYPSER, Andreas, 68159 Mannheim (DE); RHEINHEIMER, Joachim, 67063 Ludwigshafen (DE); SCHÄFER, Peter, 67308 Ottersheim (DE); SCHIEWECK, Frank, 67258 Hessheim (DE); SCHWÖGLER, Anja, 68165 Mannheim (DE); AMMERMANN, Eberhard, 64646 Heppenheim (DE); STRATHMANN, Siegfried, 67117 Limburgerhof (DE); LORENZ, Gisela, 67434 Neustadt (DE); STIERL, Reinhard, 67251 Freinsheim (DE); SCHÖFL, Ulrich, 68782 Brühl (DE)
(74) Vertreter: Isenbruck, Günter
(86) Internationale Anmeldenummer: PCT/EP2003/002847
(87) Internationale Veröffentlichungsnummer: WO 2003/080615

(56) Entgegenhaltungen:
- EP-A- 0 394 644
- EP-A- 0 410 244
- EP-A- 0 418 175
- EP-A- 0 510 526
- WO-A-02/02563
- WO-A-02/50077
- WO-A-99/40072
- WO-A-99/41255
- WO-A-03/008417
- GB-A- 2 217 710
- BEARE, NEIL A. ET AL: "Palladium-Catalyzed Arylation of Malonates and Cyano Esters Using Sterically Hindered Trialkyl- and Ferrocenyldialkylphosphine Ligands" JOURNAL OF ORGANIC CHEMISTRY (2002), 67(2), 541-555 , XP002245228
- DATABASE CA [Online] CHEMICAL ABSTRACTS SERVICE, COLUMBUS, OHIO, US; IKENO, IKUYO: "Method for preparation of phenylacetic acid and benzonitrile derivatives" retrieved from STN Database accession no. 134:4767 XP002245232 & JP 2000 327629 A (NIPPON SHOKUBAI KAGAKU KOGYO CO., LTD., JAPAN) 28. November 2000 (2000-11-28)
- DATABASE CA [Online] CHEMICAL ABSTRACTS SERVICE, COLUMBUS, OHIO, US; DELL'ERBA, CARLO ET AL: "SRN1 arylation of active methylene compounds by arylazo sulfides in DMSO" retrieved from STN Database accession no. 128:101877 XP002245233 & GAZZETTA CHIMICA ITALIANA (1997), 127(7), 361-366 ,
- TONA, MERCE ET AL: "A study on the mechanism and scope of the radical-mediated oxidation of arylacetoacetates" TETRAHEDRON (1995), 51(36), 10041-52 , XP002245229
- TONA, MERCE ET AL: "A comparative study on the photo-induced arylation of.beta.-dicarbonyl compounds by arylazo sulfides and its use in the synthesis of methyl labeled 2-arylpropionic acids" TETRAHEDRON (1994), 50(27), 8117-26 , XP002245230
- DATABASE CA [Online] CHEMICAL ABSTRACTS SERVICE, COLUMBUS, OHIO, US; NARITA, HIROKAZU ET AL: "Preparation of quinolonecarboxylic acids and analogs as antibacterials" retrieved from STN Database accession no. 113:132028 XP002245234 & JP 02 085255 A (TOYAMA CHEMICAL CO., LTD., JAPAN) 26. März 1990 (1990-03-26)
- MOMOSE, TSUTOMU ET AL: "Organic analysis. XI. Infrared spectra of phenylsulfonyl derivatives. 1. The SO2 stretching frequencies" CHEMICAL & PHARMACEUTICAL BULLETIN (1958), 6, 415-21 , XP008018734
- DATABASE CA [Online] CHEMICAL ABSTRACTS SERVICE, COLUMBUS, OHIO, US; VLASOV, V. M. ET AL: "Mesomeric carbanions of polyfluoroarylmethanes. Fluorine-19 NMR spectra and the scale of relative stability" retrieved from STN Database accession no. 88:6108 XP002245235 & IZVESTIYA SIBIRSKOGO OTDELENIYA AKADEMII NAUK SSSR, SERIYA KHIMICHESKIKH NAUK (1977), (5), 127-36 ,
- HENNESSY ET AL.: "A general and mild copper-catalyzed arylation of diethyl malonate" ORGANIC LETTERS., Bd. 4, Nr. 2, 2002, Seiten 269-272, XP002245231 ACS, WASHINGTON, DC., US ISSN: 1523-7060

## Beschreibung

Die vorliegende Erfindung betrifft Triazolopyrimidine der Formel I, 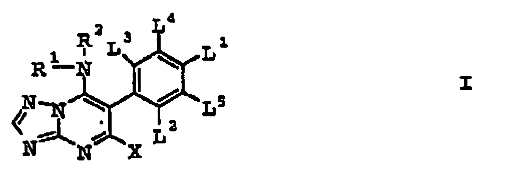 in die substituenten folgende Bedeutung haben:
- L¹: Cyano oder C(=O)A², worin
A² C₁-C₈-Alkoxy, C₁-C₆-Halogenalkoxy, Wasserstoff, Hydroxy, C₁-C₈-Alkyl, C₁-C₆-Alkylamino oder Di-(C₁-C₈-Alkyl)amino bedeutet;
- L²,L³: Wasserstoff oder Halogen;
- L⁴,L⁵: Wasserstoff, Halogen oder C₁-C₄-Alkyl;
- X: Halogen, Cyano, C₁-C₄-Alkyl, C₁-C₄-Halogenalkyl, C₁-C₄-Alkoxy oder C₁-C₂-Halogenalkoxy;
- R¹: C₁-C₆-Alkyl, C₁-C₆-Halogenalkyl, C₃-C₆-Cycloalkyl, C₃-C₆-Halogencycloalkyl, C₂-C₈-Alkenyl, C₂-C₈-Halogenalkenyl, C₂-C₈-Alkinyl; und
- R²: Wasserstoff oder C₁-C₄-Alkyl; oder
R¹ und R² können auch zusammen mit dem Stickstoffatom, an das sie gebunden sind, einen fünf- oder sechsgliedrigen gesättigten oder ungesättigten Ring bilden, der einen oder zwei Substituenten aus der Gruppe Halogen, C₁-C₆-Alkyl und C₁-C₈-Halogenalkyl tragen kann.

Außerdem betrifft die Erfindung Verfahren und Zwischenprodukte zur Herstellung dieser Verbindungen, sie enthaltende Mittel sowie ihre Verwendung zur Bekämpfung von Schadpilzen.

Aus EP-A 71 792, EP-A 550 113, WO-A 98/46608 und WO-A 99/41255 sind 5-Chlortriazolopyrimidine zur Bekämpfung von Schadpilzen bekannt.

Fungizid wirksame Triazolopyrimidine mit spezieller Substitution der 6-Phenylgruppe sind aus EP-A 834 513, WO 98/46607, EP-A 945 453, WO 99/48893, US 5 985 883 und WO 02/46195 bekannt.

Ihre Wirkung ist jedoch in vielen Fällen, insbesondere bei niedrigen Aufwandmengen, nicht immer zufriedenstellend.

Davon ausgehend liegt der vorliegenden Erfindung die Aufgabe zugrunde, Verbindungen mit verbesserter Wirkung und/oder verbreitertem Wirkungsspektrum bereitzustellen.

Demgemäß wurden die eingangs definierten Verbindungen gefunden. Desweiteren wurden Verfahren und Zwischenprodukte zu ihrer Herstellung, sie enthaltende Mittel sowie Verfahren zur Bekämpfung von Schadpilzen unter Verwendung der Verbindungen I gefunden.

Die Verbindungen der Formel I unterscheiden sich von den aus den oben genannten Schriften in der Substitution der 6-Phenylgruppe, die in der para-Position eine Gruppe L¹ trägt und der Substitution der 7-Amino-Gruppe.

Die Verbindungen der Formel I weisen eine gegenüber den bekannten Verbindungen erhöhte Wirksamkeit gegen Schadpilze auf.

Die erfindungsgemäßen Verbindungen können auf verschiedenen Wegen erhalten werden. Vorteilhaft werden sie durch Umsetzung von 5-Aminotriazol der Formel II mit entsprechend substituierten Phenylmalonaten der Formel III, in der R für Alkyl, bevorzugt für C₁-C₆-Alkyl, insbesondere für Methyl oder Ethyl steht, dargestellt.

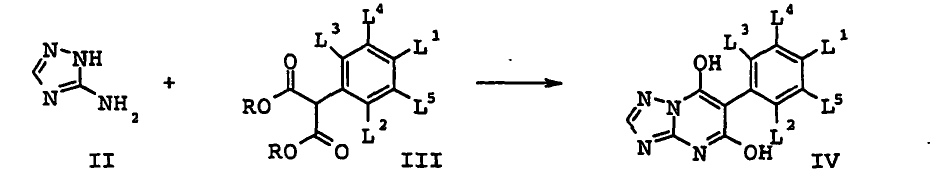

Diese Umsetzung erfolgt üblicherweise bei Temperaturen von 80°C bis 250°C, vorzugsweise 120°C bis 180°C, ohne Solvens oder in einem inerten organischen Lösungsmittel in Gegenwart einer Base [vgl. EP-A 770 615] oder in Gegenwart von Essigsäure unter den aus Adv. Het. Chem. Bd. 57, S. 81ff. (1993) bekannten Bedingungen.

Geeignete Lösungsmittel sind aliphatische Kohlenwasserstoffe, aromatische Kohlenwasserstoffe wie Toluol, o-, m- und p-Xylol, halogenierte Kohlenwasserstoffe, Ether, Nitrile, Ketone, Alkohole, sowie N-Methylpyrrolidon, Dimethylsulfoxid, Dimethylformamid und Dimethylacetamid. Besonders bevorzugt wird die Umsetzung ohne Lösungsmittel oder in Chlorbenzol, Xylol, Dimethylsulfoxid, N-Methylpyrrolidon durchgeführt. Es können auch Gemische der genannten Lösungsmittel verwendet werden.

Als Basen kommen allgemein anorganische Verbindungen wie Alkalimetall- und Erdalkalimetallhydroxide, Alkalimetall- und Erdalkalimetalloxide, Alkalimetall- und Erdalkalimetallhydride, Alkalimetallamide, Alkalimetall- und Erdalkalimetallcarbonate sowie Alkalimetallhydrogencarbonate, metallorganische Verbindungen, insbesondere Alkalimetallalkyle, Alkylmagnesiumhalogenide sowie Alkalimetall- und Erdalkalimetallalkoholate und Dimethoxymagnesium, außerdem organische Basen, z.B. tertiäre Amine wie Trimethylamin, Triethylamin, Tri-isopropylethylamin, Tributylamin und N-Methylpiperidin, N-Methylmorpholin, Pyridin, substituierte Pyridine wie Collidin, Lutidin und 4-Dimethylaminopyridin sowie bicyclische Amine in Betracht. Besonders bevorzugt werden tertiäre amine wie Tri-isopropylethylamin, Tributylamin, N-Methylmorpholin oder N-Methylpiperidin.

Die Basen werden im allgemeinen in katalytischen Mengen eingesetzt, sie können aber auch äquimolar, im Überschuß oder gegebenenfalls als Lösungsmittel verwendet werden.

Die Edukte werden im allgemeinen in äquimolaren Mengen miteinander umgesetzt. Es kann für die Ausbeute vorteilhaft sein, die Base und das Malonat III in einem Überschuß bezogen auf das Triazol einzusetzen.

Phenylmalonate der Formel III werden vorteilhaft aus der Reaktion entsprechend substituierter Brombenzole mit Dialkylmalonaten unter Cu(I)-Katalyse erhalten [vgl. Chemistry Letters, S. 367-370, 1981; EP-A 10 02 788].

Die Dihydroxytriazolopyrimidine der Formel IV werden unter den aus WO-A 94/20501 bekannten Bedingungen in die Dihalogenpyrimidine der Formel V überführt. Als Halogenierungsmittel [Hal] wird vorteilhaft ein Chlorierungsmittel oder ein Bromierungsmittel, wie Phosphoroxybromid oder Phosphoroxychlorid, ggf. in Anwesenheit eines Lösungsmittels, eingesetzt.

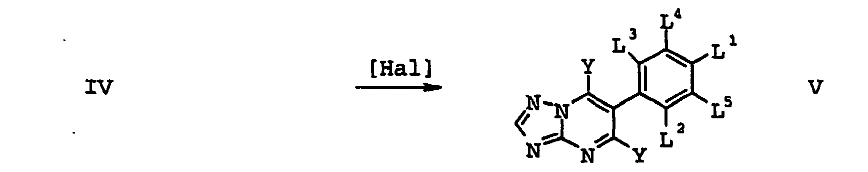

Diese Umsetzung wird üblicherweise bei 0°C bis 150°C, bevorzugt bei 80°C bis 125°C, durchgeführt [vgl. EP-A 770 615].

Dihalogenpyrimidine der Formel V werden mit Aminen der Formel VI, 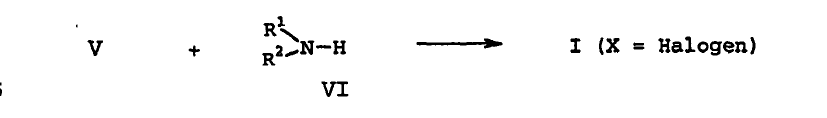 in der R¹ und R² wie in Formel I definiert sind, zu Verbindungen der Formel I, in der X für Halogen steht, weiter umgesetzt.

Diese Umsetzung wird vorteilhaft bei 0°C bis 70°C, bevorzugt 10°C bis 35°C durchgeführt, vorzugsweise in Anwesenheit eines inerten Lösungsmittels, wie Ether, z. B. Dioxan, Diethylether oder insbesondere Tetrahydrofuran, halogenierte Kohlenwasserstoffe, wie Dichloromethan und aromatische Kohlenwasserstoffe, wie beispielsweise Toluol [vgl. WO-A 98/46608].

Die Verwendung einer Base, wie tertiäre Amine, beispielsweise Triethylamin oder anorganische Amine, wie Kaliumcarbonat ist bevorzugt; auch überschüssiges Amin der Formel VI kann als Base dienen.

Verbindungen der Formel I, in der X Cyano, C₁-C₆-Alkoxy oder C₁-C₂-Halogenalkoxy bedeutet, können vorteilhaft aus der Umsetzung von Verbindungen I, in der X Halogen, bevorzugt Chlor bedeutet, mit Verbindungen M-X' (Formel VII) erhalten werden. Verbindungen VII stellen je nach der Bedeutung der einzuführenden Gruppe X' ein anorganisches Cyanid, ein Alkoxylat oder ein Halogenalkoxylat dar. Die Umsetzung erfolgt vorteilhaft in Anwesenheit eines inerten Lösungsmittels. Das Kation M in Formel VII hat geringe Bedeutung; aus praktischen Gründen sind üblicherweise Ammonium-, Tetraalkylanunonium- oder Alkali- oder Erdalkalimetallsalze bevorzugt. 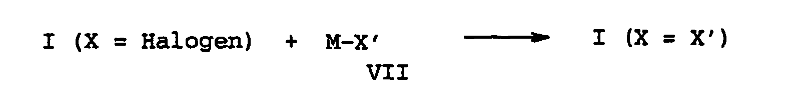 Üblicherweise liegt die Reaktionstemperatur bei 0 bis 120°C, bevorzugt bei 10 bis 40°C [vgl. J. Heterocycl. Chem., Bd.12, S. 861-863 (1975)].

Geeignete Lösungsmittel umfassen Ether, wie Dioxan, Diethylether und, bevorzugt Tetrahydrofuran, halogenierte Kohlenwasserstoffe wie Dichloromethan und aromatische Kohlenwasserstoffe, wie Toluol.

Verbindungen der Formel I, in denen X für C₁-C₄-Alkyl oder C₁-C₄-Halogenalkyl steht, können vorteilhaft durch folgenden Syntheseweg erhalten werden: 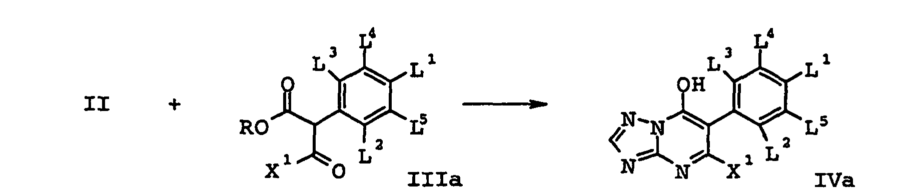 Ausgehend von den Ketoestern IIIa werden die 5-Alkyl-7-hydroxy-6-phenyltriazolopyrimidine IVa erhalten. Durch Verwendung der leicht zugänglichen 2-Phenylacetessigestern (IIIa mit X¹=CH₃) werden die 5-Methyl-7-hydroxy-6-phenyltriazolopyrimidine erhalten [vgl. Chem. Pharm. Bull., 9, 801, (1961)]. Die Herstellung der Ausgangsverbindungen IIIa erfolgt vorteilhaft unter den aus EP-A 10 02 788 beschrieben Bedingungen.

Die so erhaltenen 5-Alkyl-7-hydroxy-6-phenyltriazolopyrimidine werden mit Halogenierungsmitteln [Hal] zu den 7-Halogenotriazolopyrimidinen der Formel Va umgesetzt. Bevorzugt werden Chlorierungs- oder Bromierungsmittel wie Phosphoroxybromid, Phosphoroxychlorid, Thionylchlorid, Thionylbromid oder Sulfurylchlorid eingesetzt. Die Umsetzung kann in Substanz oder in Gegenwart eines Lösungsmittels durchgeführt werden. Übliche Reaktionstemperaturen betragen von 0 bis 150°C oder vorzugsweise von 80 bis 125°C. 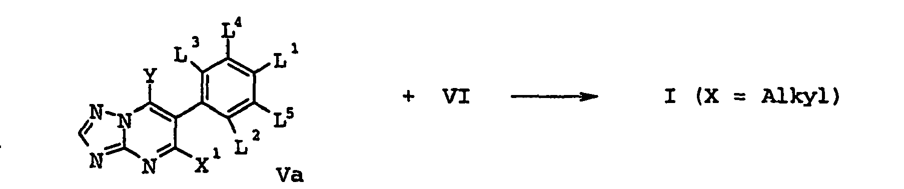 Die Umsetzung von Va mit Aminen VI erfolgt unter den weiter oben beschriebenen Bedingungen.

Verbindungen der Formel I in der X C₁-C₄-Alkyl bedeutet, können alternativ auch aus Verbindungen I, in der X Halogen, insbesondere Chlor, bedeutet und Malonaten der Formel VIII hergestellt werden. In Formel VIII bedeuten X" Wasserstoff oder C₁-C₃-Alkyl und R C₁-C₄-Alkyl. Sie werden zu Verbindungen der Formel IX umgesetzt und zu Verbindungen I decarboxyliert [vgl. US 5,994,360]. 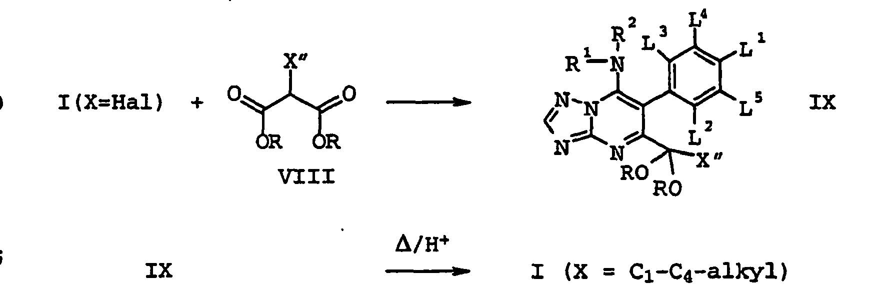 Die Malonate VIII sind in der Literatur bekannt [J. Am. Chem. Soc., Bd. 64, 2714 (1942); J. Org. Chem., Bd. 39, 2172 (1974); Helv. Chim. Acta, Bd. 61, 1565 (1978)] oder können gemäß der zitierten Literatur hergestellt werden.

Die anschließende Verseifung des Esters IX erfolgt unter allgemein üblichen Bedingungen, in Abhängigkeit der verschiedenen Strukturelemente kann die alkalische oder die saure Verseifung der Verbindungen IX vorteilhaft sein. Unter den Bedingungen der Esterverseifung kann die Decarboxylierung zu I bereits ganz oder teilweise erfolgen.

Die Decarboxylierung erfolgt üblicherweise bei Temperaturen von 20°C bis 180°C, vorzugsweise 50°C bis 120°C, in einem inerten Lösungsmittel, gegebenenfalls in Gegenwart einer Säure.

Geeignete Säuren sind Salzsäure, Schwefelsäure, Phosphorsäure, Ameisensäure, Essigsäure, p-Toluolsulfonsäure. Geeignete Lösungsmittel sind Wasser, aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt wird die Reaktion in Salzsäure oder Essigsäure durchgeführt. Es können auch Gemische der genannten Lösungsmittel verwendet werden.

Verbindungen der Formel I, in denen X für C₁-C₄-Alkyl steht, können auch durch Kupplung von 5-Halogentriazolopyrimidinen der Formel I, in der X Halogen bedeutet, mit metallorganischen Reagenzien der Formel X erhalten werden. In einer Ausführungsform dieses Verfahrens erfolgt die Umsetzung unter Übergangsmetallkatalyse, wie Ni- oder Pd-Katalyse.

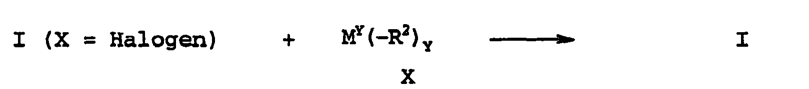

In Formel X steht M für ein Metallion der Wertigkeit Y, wie beispielsweise B, Zn oder Sn. Diese Reaktion kann beispielsweise analog folgender Methoden durchgeführt werden: J. Chem. Soc. Perkin Trans. 1, 1187 (1994), ebenda 1, 2345 (1996); WO-A 99/41255; Aust. J. Chem., Bd. 43, 733 (1990); J. Org. Chem., Bd. 43, 358 (1978); J. Chem. Soc. Chem. Commun. 866 (1979); Tetrahedron Lett., Bd. 34, 8267 (1993); ebenda, Bd. 33, 413 (1992).

Sofern R¹ und R² halogenfreie Gruppen darstellen, sind optisch aktive Amine der Formel VI in der (R)-Konfiguration bevorzugt.

Sofern R¹ oder R² Halogenalkyl oder Halogenalkenylgruppen beinhalten ist für optisch aktive Amine der Formel VI die (S)-Konfiguration bevorzugt.

Amine der Formel VI sind entweder kommerziell erhältlich oder, sofern sie ein Chiralitätszentrum aufweisen, über Racematspaltung gemäß WO 02/38565 zugänglich. Besonders vorteilhaft läßt sich beispielsweise (R)-3,3-Dimethyl-but-2-amin (R-DMBA) auf diesem Weg herstellen.

In einer bevorzugten Ausführungsform des erfindungsgemäßen Verfahrens zur Herstellung der Verbindungen, in denen L¹ S(=O)ₙA¹ mit n = 1 oder 2 bedeutet, erfolgt die Oxidation der Thioverbindungen mit n = 0 auf der Stufe der Formel I [Lit.: WO 94/14761; Synth. Commun. Bd.16, S.233 (1986)].

Verbindungen der Formeln I, III und IV, in denen L¹ für S(=O)ₙA¹ mit n = 2 und A¹ Alkyl, insbesondere Methyl steht, können auch als Zwischenprodukte zur Herstellung weiterer 6-Phenyltriazolopyrimidine verwendet werden. Diese Zwischenprodukte sind zur Herstellung solcher Triazolopyrimidine vorteilhaft zu verwenden, in denen die 6-Phenylgruppe in para-Stellung eine als Nucleophil einführbare Gruppe, wie beispielsweise Cyano, Nitro, Hydroxy, Alkoxy, Halogenalkoxy oder über Stickstoff gebundene Gruppen, wie Alkylamino, Dialkylamino oder einen über N gebundenen Heterocyclus, aufweisen [Lit.: Tetrahedron Lett. S.759 (1967); ebenda S.1763 (2000); J. Org. Chem. S. 4705 (1979)]. Besonders vorteilhaft findet der Austausch der SO₂-Alkylgruppe gegen den einzuführenden Substituenten auf der Stufe der Formel 1 statt.

Verbindungen der Formel I, in denen L¹ für C(=O)A² mit A² = Wasserstoff oder Alkyl steht, werden vorteilhaft aus den entsprechenden Verbindungen, in denen L¹ für CN steht, hergestellt. Besonders bevorzugt erfolgt diese Umsetzung auf der Stufe der Formel I.

Die Herstellung von Verbindungen, in denen L¹ CHO bedeutet, erfolgt bevorzugt aus den entsprechenden Cyaniden durch Reduktion unter bekannten Bedingungen [vgl. Collect. Czech. Chem. Commun., S.729 (2000); J. Org. Chem., S.5298 (2000); Heterocycles, S.1173 (1987); Chem. Pharm. Bull., S.1440 (1991)]. Verbindungen, in denen L¹ C(O)Alkyl bedeutet, werden vorteilhaft aus den entsprechenden Cyaniden durch Umsetzung mit Grignard- oder Alkyllithium-Verbindungen unter bekannten Bedingnugen erhalten [vgl. J. Org. Chem., S.4844 (1994); Synthetic Commun., S.4067 (1998); Tetrahedron Lett., S.6505 (1988)]

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form farbloser oder schwach bräunlicher, zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Sofern einzelne Verbindungen I nicht auf den voranstehend beschriebenen Wegen zugänglich sind, können sie durch Derivatisierung anderer Verbindungen I hergestellt werden.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz erfolgen.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom und Jod;
**Alkyl:** gesättigte, geradkettige oder verzweigte Kohlenwasserstoffreste mit 1 bis 4, 6 oder 8 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
**Halogenalkyl:** geradkettige oder verzweigte Alkylgruppen mit 1 bis 8 Kohlenstoffatomen (wie vorstehend genannt), wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl', 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl oder 1,1,1-Trifluorprop-2-yl;
**Alkenyl:** ungesättigte, geradkettige oder verzweigte Kohlenwasserstoffreste mit 2 bis 4, 6 oder 8 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-lpropenyl und 1-Ethyl-2-methyl-2-propenyl;
**Halogenalkenyl:** ungesättigte, geradkettige oder verzweigte Kohlenwasserstoffreste mit 2 bis 8 Kohlenstoffatomen und einer Doppelbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Alkinyl:** geradkettige oder verzweigte Kohlenwasserstoffgruppen mit 2 bis 4, 6 oder 8 Kohlenstoffatomen und einer Dreifachbindung in einer beliebigen Position, z.B. C₂-C₆-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
**Halogenalkinyl:** ungesättigte, geradkettige oder verzweigte Kohlenwasserstoffreste mit 2 bis 8 Kohlenstoffatomen und einer Dreifachbindung in einer beliebigen Position (wie vorstehend genannt), wobei in diesen Gruppen die Wasserstoffatome teilweise oder vollständig gegen Halogenatome wie vorstehend genannt, insbesondere Fluor, Chlor und Brom, ersetzt sein können;
**Cycloalkyl:** mono- oder bicyclische, gesättigte Kohlenwasserstoffgruppen mit 3 bis 6 oder 8 Kohlenstoffringgliedern, z.B. C₃-C₈-Cycloalkyl wie Cyclopropyl, Cyclobutyl, Cyclopentyl, Cyclohexyl, Cycloheptyl und Cyclooctyl;
**Alkoxycarbonyl:** eine Alkoxygruppe mit 1 bis 6 Kohlenstoffatomen (wie vorstehend genannt), welche über eine Carbonylgruppe (-CO-) an das Gerüst gebunden ist;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O;
**fünf- bis zehngliedriger gesättigter, partiell ungesättigter oder aromatischer Heterocyclus, enthaltend ein bis vier Heteroatome aus der Gruppe O, N oder S:**
   - **5- oder 6-gliedriges Heterocyclyl,** enthaltend ein bis drei Stickstoffatome und/oder ein Sauerstoff- oder Schwefelatom oder ein oder zwei Sauerstoff- und/oder Schwefelatome, z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 2-Pyrrolin-2-yl, 2-Pyrrolin-3-yl, 3-Pyrrolin-2-yl, 3-Pyrrolin-3-yl, 2-Isoxazolin-3-yl, 3-Isoxazolin-3-yl, 4-Isoxazolin-3-yl, 2-Isoxazolin-4-yl, 3-Isoxazolin-4-yl, 4-Isoxazolin-4-yl, 2-Isoxazolin-5-yl, 3-Isoxazolin-5-yl, 4-Isoxazolin-5-yl, 2-Isothiazolin-3-yl, 3-Isothiazolin-3-yl, 4-Isothiazolin-3-yl, 2-Isothiazolin-4-yl, 3-Isothiazolin-4-yl, 4-Isothiazolin-4-yl, 2-Isothiazolin-5-yl, 3-Isothiazolin-5-yl, 4-Isothiazolin-5-yl, 2,3-Dihydropyrazol-1-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-1-yl, 3,4-Dihydropyrazol-3-yl, 3,4-Dihydropyrazol-4-yl, 3,4-Dihydropyrazol-5-yl, 4,5-Dihydropyrazol-1-yl, 4,5-Dihydropyrazol-3-yl, 4,5-Dihydropyrazol-4-yl, 4,5-Dihydropyrazol-5-yl, 2,3-Dihydrooxazol-2-yl, 2,3-Dihydrooxazol-3-yl, 2,3-Dihydrooxazol-4-yl, 2,3-Dihydrooxazol-5-yl, 3,4-Dihydrooxazol-2-yl, 3,4-Dihydrooxazol-3-yl, 3,4-Dihydrooxazol-4-yl, 3,4-Dihydrooxazol-5-yl, 3,4-Dihydrooxazol-2-yl, 3,4-Dihydrooxazol-3-yl, 3,4-Dihydrooxazol-4-yl, 2-Piperidinyl, 3-Piperidinyl, 4-Piperidinyl, 1,3-Dioxan-5-yl, 2-Tetrahydropyranyl, 4-Tetrahydropyranyl, 2-Tetrahydrothienyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydro-triazin-2-yl und 1,2,4-Hexahydrotriazin-3-yl;
   - **5-gliedriges Heteroaryl,** enthaltend ein bis vier Stickstoffatome oder ein bis drei Stickstoffatome und ein Schwefel- oder Sauerstoffatom: 5-Ring Heteroarylgruppen, welche neben Kohlenstoffatomen ein bis vier Stickstoffatome oder ein bis drei Stickstoffatome und ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   - **6-gliedriges Heteroaryl,** enthaltend ein bis drei bzw. ein bis vier Stickstoffatome: 6-Ring Heteroarylgruppen, welche neben Kohlenstoffatomen ein bis drei bzw: ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl;
**Alkylen:** divalente unverzweigte Ketten aus 3 bis 5 CH₂-Gruppen, z.B. CH₂, CH₂CH₂, CH₂CH₂CH₂, CH₂CH₂CH₂CH₂ und CH₂CH₂CH₂CH₂CH₂;
**Oxyalkylen:** divalente unverzweigte Ketten aus 2 bis 4 CH₂-Gruppen, wobei eine Valenz über ein Sauerstoffatom an das Gerüst gebunden ist, z.B. OCH₂CH₂, OCH₂CH₂CH₂ und OCH₂CH₂CH₂CH₂;
**Oxyalkylenoxy:** divalente unverzweigte Ketten aus 1 bis 3 CH₂-Gruppen, wobei beide Valenzen über ein Sauerstoffatom an das Gerüst gebunden ist, z.B. OCH₂O, OCH₂CH₂O und OCH₂CH₂CH₂O.

In dem Umfang der vorliegenden Erfindung sind die (R) - und (8)-Isomere und die Razemate von Verbindungen der Formel I eingeschlossen, die chirale Zentren aufweisen.

Die besonders bevorzugten Ausführungsformen der Zwischenprodukte in Bezug auf die Variablen entsprechen denen der Reste L¹, L², L³, L⁴, L⁵, R¹, R² und X der Formel I.

Im Hinblick auf ihre bestimmungsgemäße Verwendung der Triazolopyrimidine der Formel I sind die folgenden Bedeutungen der Substituenten, und zwar jeweils für sich allein oder in Kombination, besonders bevorzugt:

Verbindungen I werden bevorzugt, in denen R¹ für C₁-C₆-Alkyl oder C₁-C₈-Halogenalkyl steht.

Daneben werden Verbindungen I bevorzugt, in denen R¹ für C₂-C₁₀-Alkenyl oder C₂-C₁₀-Alkinyl steht.

Verbindungen I sind besonders bevorzugt, in denen R¹ für eine Gruppe B steht 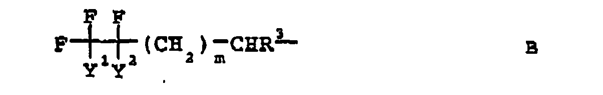 worin
- Y¹: Wasserstoff, Fluor oder C₁-C₆-Fluoroalkyl,
- Y²: Wasserstoff oder Fluor, oder Y¹ und Y² bilden gemeinsam eine Doppelbindung;
- m: is 0 oder 1; und
- R³: Wasserstoff oder Methyl bedeuten.

Außerdem werden Verbindungen I bevorzugt, in denen R¹ für C₃-C₆-cycloalkyl steht, welches durch C₁-C₄-Alkyl substituiert sein kann.

Insbesondere werden Verbindungen 1 bevorzugt, in denen R² Wasserstoff bedeutet.

Gleichermaßen bevorzugt sind Verbindungen I, in denen R² für Methyl oder Ethyl steht.

Sofern R¹ und/oder R² halogenfreie Gruppen mit Chiralitätszentrum darstellen, sind die (R)-Isomere bevorzugt. Sofern R¹ und/oder R² Halogenalkyl oder Halogenalkenylgruppen mit Chiralitätszentrum beinhalten, sind die (S)-Isomere bevorzugt.

Weiterhin werden Verbindungen I besonders bevorzugt, in denen R¹ und R² zusammen mit dem Stickstoffatom, an das sie gebunden sind, einen fünf- oder sechsgliedrigen Ring bilden, der einer oder zwei Substituenten aus der Gruppe Halogen, C₁-C₆-Alkyl, C₁-C₆-Halogenalkyl, tragen kann.

Besonders bevorzugt sind Verbindungen I, in denen R¹ und R² zusammen mit dem Stickstoffatom, an das sie gebunden sind, einen fünf- oder sechsgliedrigen Ring bilden, der ggf. eine Doppelbindung aufweisen kann und wie voranstehend beschrieben substituiert sein kann.

Insbesondere werden Verbindungen I besonders bevorzugt, in denen R¹ und R² zusammen mit dem Stickstoffatom, an das sie gebunden sind, einen Piperidin-, bilden, der ggf. durch eine bis zwei Gruppen Halogen, C₁-C₄-Alkyl oder C₁-C₄-Halogenalkyl, insbesondere durch 4-Methyl substituiert ist.

Weiterhin besonders bevorzugt sind Verbindungen I, in denen R¹ und R² zusammen mit dem Stickstoffatom, an das sie gebunden sind, einen Pyrrolidinring bilden, der ggf. durch eine oder zwei Gruppen Halogen, C₁-C₄-Alkyl oder C₁-C₄-Halogenalkyl, insbesondere durch 2-Methyl substituiert ist.

Bevorzugt sind Verbindungen der Formel I, in der mindestens eine Gruppe L² und/oder L³ nicht Wasserstoff bedeutet.

Eine weitere bevorzugte Ausgestaltung der Verbindungen der Formel I sind solche, in der L¹ Cyano oder C(=O)A² bedeuten. Sie werden als Verbindungen I.2 bezeichnet.

Außerdem werden Verbindungen I.2 besonders bevorzugt, in denen L¹ C₁-C₆-Alkoxycarbonyl bedeutet.

Gleichermaßen besonders bevorzugt sind Verbindungen I.2, in denen L² Halogen und L³ Halogen oder Wasserstoff, insbesondere Halogen, bedeuten.

Daneben werden Verbindungen 1.2 besonders bevorzugt, in denen L⁴ Wasserstoff und L⁵ Wasserstoff oder Methyl bedeuten.

Insbesondere werden Verbindungen der Formeln IA und IB bevorzugt, in denen die Variablen die für Formel 1 gegebene Bedeutung besitzen:

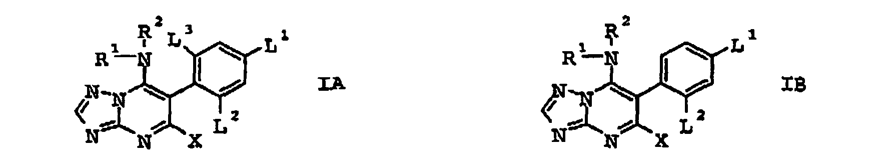

Weiterhin bevorzugt werden Verbindungen IC:

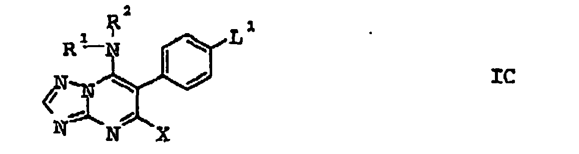

Daneben werden auch Verbindungen I.2 bevorzugt, in denen L¹ C(=0)OCH₃, L² Fluor, L³ und L⁵ Wasserstoff und L⁴ Methyl bedeuten.

Verbindungen I werden besonders bevorzugt, in denen X Chlor bedeutet.

Insbesondere sind im Hinblick auf ihre Verwendung die in den folgenden Tabellen zusamnengestellten Verbindungen I bevorzugt. Die in den Tabellen für einen Substituenten genannten Gruppen stellen außerdem für sich betrachtet, unabhängig von der Kombination, in der sie genannt sind, eine besonders bevorzugte Ausgestaltung des betreffenden Substituenten dar.

### Tabelle 10

Verbindungen der Formel IA, in denen X für Chlor, L¹ für C(=O)OCH₃ und L² und L³ Fluor stehen und die Kombination von R¹ und R² für eine Verbindung jeweils einer Zeile der Tabelle A entspricht

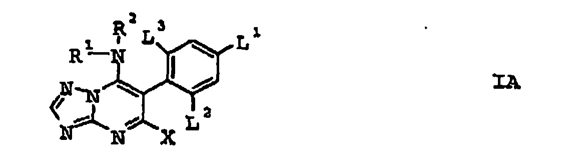

### Tabelle 11

Verbindungen der Formel IA, in denen X für Chlor, L¹ für Cyano und L² und L³ Fluor stehen und die Kombination von R¹ und R² für eine Verbindung jeweils einer Zeile der Tabelle A entspricht

### Tabelle 12

Verbindungen der Formel IA, in denen X für Chlor, L¹ für CHO und L² und L³ Fluor stehen und die Kombination von R¹ und R² für eine Verbindung jeweils einer Zeile der Tabelle A entspricht

### Tabelle 13

Verbindungen der Formel IA, in denen X für Chlor, L¹ für C(=O)CH₃ und L² und L³ Fluor stehen und die Kombination von R¹ und R² für eine Verbindung jeweils einer Zeile der Tabelle A entspricht

### Tabelle 14

Verbindungen der Formel IA, in denen X für Chlor, L² für C(=O)NHCH₃ und L² und L³ stehen und die Kombination von R¹ und R² für eine Verbindung jeweils einer Zeile der Tabelle A entspricht

### Tabelle 15

Verbindungen der Formel IB, in denen X für Chlor, L¹ für C(=O)OCH₃ und L² für Fluor steht und die Kombination von R¹ und R² für eine Verbindung jeweils einer Zeile der Tabelle A entspricht

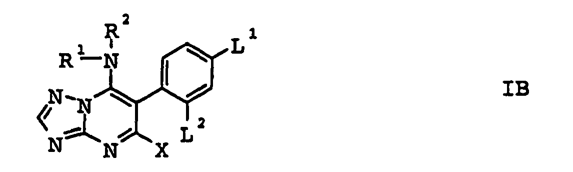

### Tabelle 16

Verbindungen der Formel IB, in denen X für Chlor, L¹ für C(=O)OCH₃ und L² für Chlor steht und die Kombination von R¹ und R² für eine Verbindung jeweils einer Zeile der Tabelle A entspricht

### Tabelle 17

Verbindungen der Formel IC, in denen X für Chlor und L¹ für C(=O)OCH₃ steht und die Kombination von R¹ und R² für eine Verbindung jeweils einer Zeile der Tabelle A entspricht

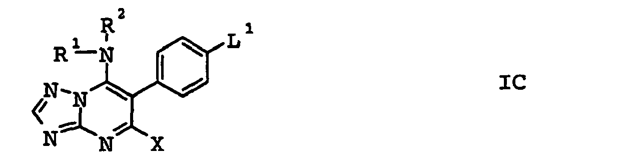

### Tabelle 18

Verbindungen der Formel IC, in denen X für Chlor und L¹ für Cyano steht und die Kombination von R¹ und R² für eine Verbindung jeweils einer Zeile der Tabelle A entspricht

### Tabelle 19

Verbindungen der Formel I, in denen X für Chlor und L¹ für C(=O)OCH₃, L², L³, L⁴ und L⁵ für Fluor stehen und die Kombination von R¹ und R² für eine Verbindung jeweils einer Zeile der Tabelle A entspricht

**Tabelle A**

| **No.** | **R¹** | **R²** |
|---|---|---|
| A-1 | CH₂CH₃ | H |
| A-2 | CH₂CH₃ | CH₃ |
| A-3 | CH₂CH₃ | CH₂CH₃ |
| A-4 | CH₂CF₃ | H |
| A-5 | CH₂CF₃ | CH₃ |
| A-6 | CH₂CF₃ | CH₂CH₃ |
| A-7 | CH₂CC1₃ | H |
| A-8 | CH₂CCI₃ | CH₃ |
| A-9 | CH₂CCl₃ | CH₂CH₃ |
| A-10 | CH₂CH₂CH₃ | H |
| A-11 | CH₂CH₂CH₃ | CH₃ |
| A-12 | CH₂CH₂CH₃ | CH₂CH₃ |
| A-13 | CH₂CH₂CH₃ | CH₂CH₂CH₃ |
| A-14 | CH(CH₃)₂ | H |
| A-15 | CH(CH₃)₂ | CH₃ |
| A-16 | CH(CH₃)₂ | CH₂CH₃ |
| A-17 | (±) CH(CH₃)-CH₂CH₃ | H |
| A-18 | (±) CH(CH₃)-CH₂CH₃ | CH₃ |
| A-19 | (±) CH(CH₃)-CH₂CH₃ | CH₂CH₃ |
| A-20 | (S) CH (CH₃)-CH₂CH₃ | H |
| A-21 | (S) CH (CH₃)-CH₂CH₃ | CH₃ |
| A-22 | (S) CH(CH₃)-CH₂CH₃ | CH₂CH₃ |
| A-23 | (R) CH(CH₃)-CH₂CH₃ | H |
| A-24 | (R) CH (CH₃)-CH₂CH₃ | CH₃ |
| A-25 | (R) CH (CH₃)-CH₂CH₃ | CH₂CH₃ |
| A-26 | (±) CH(CH₃)-CH(CH₃)₂ | H |
| A-27 | (±) CH(CH₃)-CH(CH₃)₂ | CH₃ |
| A-28 | (±) CH(CH₃)-CH(CH₃)₂ | CH₂CH₃ |
| A-29 | (S) CH(CH₃)-CH(CH₃)₂ | H |
| A-30 | (S) CH(CH₃)-CH(CH₃)₂ | CH₃ |
| A-31 | (S) CH(CH₃)-CH(CH₃)₂ | CH₂CH₃ |
| A-32 | (R) CH(CH₃)-CH(CH₃)₂ | H |
| A-33 | (R) CH(CH₃)-CH(CH₃)₂ | CH₃ |
| A-34 | (R) CH(CH₃)-CH(CH₃)₂ | CH₂CH₃ |
| A-35 | (±) CH(CH₃)-C(CH₃)₃ | H |
| A-36 | (±) CH(CH₃)-C(CH₃)₃ | CH₃ |
| A-37 | (±) CH(CH₃)-C(CH₃)₃ | CH₂CH₃ |
| A-38 | (S) CH(CH₃)-C(CH₃)₃ | H |
| A-39 | (S) CH(CH₃)-C(CH₃)₃ | CH₃ |
| A-40 | (S) CH(CH₃)-C(CH₃)₃ | CH₂CH₃ |
| A-41 | (R) CH(CH₃)-C(CH₃)₃ | H |
| A-42 | (R) CH(CH₃)-C(CH₃)₃ | CH₃ |
| A-43 | (R) CH(CH₃)-C(CH₃)₃ | CH₂CH₃ |
| A-44 | (±) CH(CH₃)-CF₃ | H |
| A-45 | (±) CH(CH₃)-CF₃ | CH₃ |
| A-46 | (±) CH(CH₃)-CF₃ | CH₂CH₃ |
| A-47 | (S) CH(CH₃)-CF₃ | H |
| A-48 | (S) CH(CH₃)-CF₃ | CH₃ |
| A-49 | (S) CH(CH₃)-CF₃ | CH₂CH₃ |
| A-50 | (R) CH(CH₃)-CF₃ | H |
| A-51 | (R) CH(CH₃)-CF₃ | CH₃ |
| A-52 | (R) CH(CH₃)-CF₃ | CH₂CH₃ |
| A-53 | (±) CH(CH₃)-CCl₃ | H |
| A-54 | (±) CH(CH₃)-CCl₃ | CH₃ |
| A-55 | (±) CH(CH₃)-CCl₃ | CH₂CH₃ |
| A-56 | (S) CH(CH₃)-CCl₃ | H |
| A-57 | (S) CH (CH₃) -CCl₃ | CH₃ |
| A-58 | (S) CH (CH₃) -CCl₃ | CH₂CH₃ |
| A-59 | (R) CH(CH₃)-CCl₃ | H |
| A-60 | (R) CH(CH₃)-CCl₃ | CH₃ |
| A-61 | (R) CH (CH₃) -CCl₃ | CH₂CH₃ |
| A-62 | CH₂CF₂CF₃ | H |
| A-63 | CH₂CF₂CF₃ | CH₃ |
| A-64 | CH₂CF₂CF₃ | CH₂CH₃ |
| A-65 | CH₂(CF₂)₂CF₃ | H |
| A-66 | CH₂(CF₂)₂CF₃ | CH3 |
| A-67 | CH₂(CF₂)₂CF₃ | CH₂CH₃ |
| A-68 | CH₂C(CH₃)=CH₂ | H |
| A-69 | CH₂C(CH₃)=CH₂ | CH₃ |
| A-70 | CH₂C(CH₃)=CH₂ | CH₂CH₃ |
| A-71 | CH₂CH=CH₂ | H |
| A-72 | CH₂CH=CH₂ | CH₃ |
| A-73 | CH₂CH=CH₂ | CH₂CH₃ |
| A-74 | CH(CH₃)CH=CH₂ | H |
| A-75 | CH(CH₃)CH=CH₂ | CH₃ |
| A-76 | CH(CH₃)CH=CH₂ | CH₂CH₃ |
| A-77 | CH(CH₃)C(CH₃)=CH₂ | H |
| A-78 | CH(CH₃)C(CH₃)=CH₂ | CH₃ |
| A-79 | CH(CH₃)C(CH₃)=CH₂ | CH₂CH₃ |
| A-80 | Cyclopentyl | H |
| A-81 | Cyclopentyl | CH₃ |
| A-82 | Cyclopentyl | CH₂CH₃ |
| A-83 | Cyclohexyl | H |
| A-84 | Cyclohexyl | CH₃ |
| A-85 | Cyclohexyl | CH₂CH₃ |
| A-86 | (CH₂)₂CH=CHCH₂- | |
| A-87 | -(CH₂)₂C(CH₃)=CHCH₂- | |
| A-88 | -(CH₂)₂CH(CH₃)(CH₂)₂- | |
| A-89 | -(CH₂)₂CHF(CH₂)₂- | |
| A-90 | -(CH₂)₃CHFCH₂- | |
| A-91 | -(CH₂)₂CH(CF₃)(CH₂)₂- | |
| A-94 | -(CH₂)₅- | |
| A-95 | -(CH₂)₄- | |
| A-96 | -CH₂CH=CHCH₂- | |
| A-97 | -CH(CH₃)(CH₂)₃- | |
| A-98 | -CH₂CH(CH₃)(CH₂)₂- | |

Die Verbindungen I eignen sich als Fungizide. Sie zeichnen sich durch eine hervorragende Wirksamkeit gegen ein breites Spektrum von pflanzenpathogenen Pilzen, insbesondere aus der Klasse der *Ascomyceten, Deuteromyceten, Phycomyceten* und *Basidiomyceten,* aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Bananen, Baumwolle, Soja, Kaffee, Zuckerrohr, Wein, Obst- und Zierpflanzen und Gemüsepflanzen wie Gurken, Bohnen, Tomaten, Kartoffeln und Kürbisgewächsen, sowie an den Samen dieser Pflanzen.

Speziell eignen sie sich zur Bekämpfung folgender Pflanzenkrankheiten:
- *Alternaria-*Arten an Gemüse und Obst,
- *Botrytis cinerea* (Grauschimmel) an Erdbeeren, Gemüse, Zierpflanzen und Reben,
- *Cercospara arachidicola an Erdnüssen,*
- *Erysiphe cichoracearum* und *Sphaerotheca fuliginea* an Kürbingewächsen.
- *Blumeria graminis* (echter Mehltau) an Getreide,
- *Fusarium-* und *Vereicillium-*Arten an verschiedenen Pflanzen,
- *Helmintbosporium*-Arten an Getreide,
- *Mycosphaerella-*Arten an Bananen und Erdnüssen,
- *Phytophthora infestans* an Kartoffeln und Tomaten,
- *Plasmopara viticola* an Reben,
- *Podosphaera leucotricha* an Äpfeln,
- *Pseudocercosporella herpotrichoides* an Weizen und Gerste,
- *Pseudoperonospora*-Arten an Hopfen und Gurken,
- *Puccinia*-Arten an Getreide,
- *Pyricularia oryzae* an Reis,
- *Rhizoctonia*-Arten an Baumwolle, Reis und Rasen,
- *Septoria* nodorum an Weizen,
- *Uncinula necator* an Reben,
- *Ustilago*-Arten an Getreide und Zuckerrohr, sowie
- *Venturia*-Arten (Schorf) an Äpfeln und Birnen.

Die Verbindungen I eignen sich außerdem zur Bekämpfung von Schadpilzen wie Paecilomyces variotii im Materialschutz (z.B. Holz, . Papier, Dispersionen für den Anstrich, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung kann sowohl vor als auch nach der Infektion der Materialien, Pflanzen oder Samen durch die Pilze erfolgen.

Die fungiziden Mittel enthalten im allgemeinen zwischen 0,1 und 95, vorzugsweise zwischen 0,5 und 90 Gew.-% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die Verbindungen I können in die üblichen Formulierungen überführt werden, z.B. Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B.Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B.

Dimethylformamid, Dimethylsulfoxid, N-Methylpyrrolidon, Wasser, in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,01 und 95 Gew.-%, vorzugsweise zwischen 0,1 und 90 Gew.-% des Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.-% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.-%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.-%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16 Gew.-%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.-%).
VI. Man vermischt 90 Gew.-Teile einer erfindungsgemäßen Verbindung mit 10 Gew.-Teilen N-Methyl-α-pyrrlidon und erhält eine Lösung, die zur Anwendung in Form kleinster Tropfen geeignet ist (Wirkstoffgehalt 90 Gew.-%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.-% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die 0,1 Gew.-% des Wirkstoffs enthält.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Die Wirkstoffkonzeritrationen in den anwendungsfertigen Zubereitungen können in größeren Bereichen variiert werden. Im allgemeinen liegen sie zwischen 0,0001 und 10%, vorzugsweise zwischen 0,01 und. 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

Die erfindungsgemäßen Mittel können in der Anwendungsform als Fungizide auch zusammen mit anderen Wirkstoffen vorliegen, der z.B.. mit Herbiziden, Insektiziden, Wachstumsregulatoren, Fungiziden oder auch mit Düngemitteln. Beim Vermischen der Verbindungen I bzw. der sie enthaltenden Mittel in der Anwendungsform als Fungizide mit anderen Fungiziden erhält man in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten'erläutern, nicht aber einschränken:
- Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zinkethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
- Nitroderivate, wie Dinitro-(1-methylheptyl)-phenylcrotonat, 2-sec-Butyl-4,6-dinitrophenyl-3.3-dimethylacrylat, 2-sec-Butyl-4,6-dinitrophenyl-isopropylcarbonat, 5-Nitro-isophthalsäure-di-isopropylester;
- heterocyclische Substanzen, wie 2-Heptadecyl-2-imidazolin-acetat, 2-Chlor-N-(4'-chlor-biphenyl-2-yl)-nicotinamid, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzim.idazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthio-phthalimid,
- N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin; 2,3-Dihydro-5-carboxanilido-6-methyl-1,4-oxathiin-4,4-dioxid, 2-Methyl-5,6-dihydro-4H-pyran-3-carbonsäure-anilid, 2-Methylfuran-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-1-(2,2,2-trichlorethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethyl-morpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, (2RS,3RS)-1-[3-(2-Chlorphenyl)-2-(4-fluorphenyl)-oxiran-2-ylmethyl]-1H-1,2,4-triazol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
- Strobilurine wie Methyl-E-methoxyimino-[α-(o-tolyloxy)-o-tolyl]acetat, Methyl-E-2-{2-[6-(2-cyanophenoxy)-pyrimidin-4-yl-oxy]-phenyl}-3-methoxyacrylat, Methyl-E-methoxyimino-[α-(2-phenoxyphenyl)]-acetamid, Methyl-E-methoxyimino-[α-(2,5-dimethylphenoxy)-o-tolyl]-acetamid, Methyl-E-2-{2-[2-trifluormethylpyridyl-6-]oxymethyl]-phenyl}3-methoxyacrylat, (E,E)-Methoximino-{2-[1-(3-trifluormethylphenyl)-ethylidenaminooxymethyl]-phenyl}-essigsäuremethylester, Methyl-N-(2-{[1-(4-chlorphenyl)-1H-pyrazol-3-yl]oxymethyl}phenyl)N-methoxy-carbamat,
- Anilinopyrimidine wie N-(4,6-Dimethylpyrimidin-2-yl)-anilin, N-[4-Methyl-6-(1-propinyl)-pyrimidin-2-yl]-anilin, N-[4-Methyl-6-cyclopropyl-pyrimidin-2-yl]-anilin,
- Phenylpyrrole wie 4-(2,2-Difluor-1,3-benzodioxol-4-yl)-pyrrol-3-carbonitril,
- Zimtsäureamide wie 3-(4-Chlorphenyl)-3-(3,4-dimethoxyphenyl)-acrylsäuremorpholid, 3-(4-Fluorphenyl)-3-(3,4-dimethoxyphenyl)-acrylsäuremorpholid, .
- sowie verschiedene Fungizide, wie Dodecylguanidinacetat, 1-(3-Brom-6-methoxy-2-methyl-phenyl)-1-(2,3,4-trimethoxy-6-methyl-phenyl)-methanon, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alanin-methylester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-(3,5-Dichlorphenyl)-5-methyl-5-methoxymethyl-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol, 5-Chlor-2-cyano-4-p-tolyl-imidazol-1-sulfonsäuredimethylamid, 3,5-Dichlor-N-(3-chlor-1-ethyl-1-methyl-2-oxo-propyl)-4-methylbenzamid.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Angaben aufgeführt.

### Beispiel 1: Herstellung von 4-cyanophenylmalonsäurediethlester

Eine Suspension von Natriumhydrid (0,51 mol) in 140 ml 1,4-Dioxan wurde bei etwa 60°C während 2 Std. mit Malonsäurediethlester (0,49 mol) versetzt. Nach weiteren 10 min Rühren wurden 0,05 mol CuBr zugesetzt. Nach 15 min wurden 0,25 mol 4-Cyanobrombenzol in 10 ml Dioxan zugegeben. Die Reaktionsmischung wurde etwa 14 Std. bei 100 °C gehalten, dann bei etwa 15°C langsam mit 35 ml 12N Salzsäure versetzt. Der Niederschlag wurde abfiltriert, das Filtrat mit Diethylether extrahiert. Nach Phasentrennung wurde die organische Phase getrocknet, dann vom Lösungsmittel befreit. Es blieben 32 g der Titelverbindung zurück.

### Beispiel 2: Herstellung von 5,7-Dihydroxy-6-(4-cyanophenyl)-[1,2,4]-triazolo[1,5-a]-pyrimidin

Eine Mischung von 14 g 3-Amino-1,2,4-triazol, 0,17 mol des Esters aus Beispiel 1 und 50 ml Tributylamin (50 ml) wurde etwa sechs Std. bei 180°C gerührt. Bei etwa 70° wurde eine Lösung von 21 g NaOH in 200 ml Wasser zugegeben und die Mischung weiter 30 min gerührt. Die organische Phase wurde abgetrennt und die wässrige Phase mit Diethylether extrahiert. Aus der wässrigen Phase fiel nach Ansäuern mit konz. Salzsäure das Produkt aus. Durch Filtration wurden 28 g der Titelverbindung erhalten.

### Beispiel 3: Herstellung von 5,7-Dichlor-6-(4-cyanophenyl)-[1,2,4]-triazolo[1,5-a]-pyrimidin

Eine Mischung von 25 g des Triazolopyrimidins aus Beispiel 2 und 50 ml POCl₃ wurde für acht Std. refluxiert, dabei destillierte etwas POCl₃ ab. Der Rückstand wurde in eine CH₂Cl₂-Wasser-Mischung gegeben, die organische Phase abgetrennt, gewaschen und getrocknet, dann vom Lösungsmittel befreit. Es wurden 23 g der Titelverbindung erhalten.

### Beispiel 4: Herstellung von 5-Chlor-6-(4-cyanophenyl)-7-isopropylamino-[1,2,4]-triazolo[1,5-a]pyrimidin [I-1]

Eine Lösung von 1,5 mmol des Produkts aus Bsp. 3 in 20 ml Dichlormethan wurde unter Rühren mit einer Lösung von 1,5 mmol Isopropylamin, 1,5 mmol Triethylamin in 10 ml Dichlormethan versetzt. Die Reaktionsmischung wurde etwa 16 Std. bei 20 bis 25°C gerührt, dann mit verd. Salzsäure gewaschen. Die organische Phase wurde abgetrennt, getrocknet und vom Lösungsmittel befreit. Nach Chromatographie an Kieselgel wurden 330 mg der Titelverbindung vom Fp. 190°C erhalten.

### Beispiel 10: Herstellung von 5-Methoxy-6-(2,6-difluor-4-cyanophenyl)-7-(4-methylpiperidin-1-yl)-[1,2,4]-triazolo[1,5-a]pyrimidin

Eine Lösung von 65 mmol der Verbindung I-5 in 400 ml waaserfr. Methanol wurde nach Versetzen mit 71,5 mmol einer 30 %igen Natriunmethanolat-Lösung bei 20 - 25°C etwa 16 Std. bei dieser Temperatur gerührt. Methanol wurde abdestilliert, der Rückstand in Dichlormethan aufgenommen, dann mit Wasser gewaschen und nach Trocknen vom Lösungsmittel befreit. Aus dem Rückstand erhielt man nach Chromatographie an Kieselgel 3,68 g der Titel Verbindung.

¹H-NMR: 8,75 (s); 7,35 (d); 3,95 (s); 3,65 (d); 2,70 (t); 1,65 (m); 1,55 (m); 1,45 (m); 0,95 (d).

### Beispiel 11: Herstellung von 5-Methyl-6-(2,6-difluor-4-cyanophenyl)-7-(4-methylpiperidin-1-yl)-[1,2,4]-triazolo[1,5-a]pyrimidin

Eine Mischung von 20 ml Diethylmalonat und 0,27 g (5,65 mmol) NaH (50%ige Dispersion in Mineralöl) in 50 ml Acetonitril wurde bei 20 - 25°C etwa 2 Std. gerührt. Zu dieser Mischung wurden 4,71 mmol der Verbindung I-5 gegeben, dann wurde auf etwa 60°C aufgeheizt und bei dieser Temperatur 20 Std. gerührt. Nach Zusatz von 50 ml wässr. NH₄Cl-Lösung wurde mit verd. HCl angesäuert. Nach Extraktion mit MTBE wurden die vereinigten organischen Phasen getrocknet und eingedampft. Der Rückstand wurde durch Chromatographie an Kieselgel gereinigt. Das Reinprodukt wurde in konz. HCl aufgenommen und und 24 Std. bei 80°C gehalten. Nach Abkühlen der Reaktionsmischung wurde durch Zusatz von wässr. NaOH-Lösung ein pH-Wert von 5 eingestellt, dann mit MTBE extrahiert. Die vereinigten organischen Phasen wurden nach Trocknen vom Lösungsmittel befreit. Aus dem Rückstand erhielt man nach Chromatographie an Kieselgel. 0,78 g der Titelverbindung.

¹H-NMR: 8,75 (s) ; 7,35 (d); 3,65 (d); 2,70 (t); 2,43 (s); 1,65 (m); 1,55 (m); 1,45 (m); 0,95 (d).

### Beispiel 12: Herstellung von 5-Chlor-6-(2,6-difluor-4-carboxaldehydphenyl)-7-(4-methylpiperidin-1-yl)-[1,2,4]-triazolo[1,5-a]pyrimidin

Eine Lösung von 1,5 mmol der Verbindung I-5 in 20 ml Dichlormethan wurde bei 0°C mit einer 1M Lösung von 1,65 mmol Diisobutylaluminiumhydrid (DIBAH) in Dichlormethan versetzt und 2 Std. bei 20 - 25°C gerührt. Diese Mischung wurde mit gesätt. NH₄Cl- und 10% HCl-Lösung versetzt, die organische Phase wurde abgetrennt und mit Wasser gewaschen. Nach Trocknen wurde das Lösungsmittel entfernt, aus dem Rückstand erhielt man nach Chromatographie an Kieselgel 0,36 g der Titelverbindung.

¹H-NMR: 10,05 (s); 8,40 (s); 7,60 (d); 3,70 (d); 2,85 (t); 1,65 (m); 1,55 (m); 1,40 (m) ; 0,95 (d).

### Beispiel 13: Herstellung von 5-Chlor-6-(2,6-difluor-4-acetylphenyl)-7-(4-methylpiperidin-1-yl)-[1,2,4]-triazolo[1,5-a]pyrimidin

Eine Lösung von 1,5 mmol der Verbindung 1-5 in 20 ml Tetrahydrofuran (THF) wurde bei 20 - 25°C mit 1,65 mmol CuBr und einer 3M Lösung von 1,65 mmol Methylmagnesiumchlorid in THF versetzt und etwa 30 min gerührt. Diese Mischung wurde mit gesätt. NH₄C1- und 10% HCl-Lösung versetzt, die organische Phase wurde abgetrennt und mit Wasser gewaschen. Nach Trocknen wurde das Lösungsmittel entfernt, aus dem Rückstand erhielt man nach Chromatographie an Kieselgel 0,22 g der Titelverbindung.

¹H-NMR: 8,40 (s); 7,65 (d); 3,70 (d); 2,80 (t); 2,70 (s); 1,70 (m); 1,55 (m); 1,40 (m); 0,98 (d).

**Tabelle I**

| | | | | | | | | | |
|---|---|---|---|---|---|---|---|---|---|
| 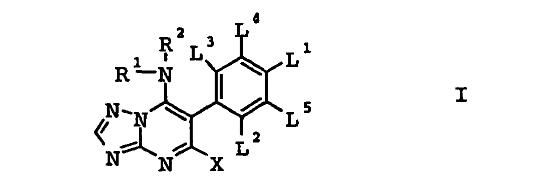 | | | | | | | | | |

| **Nr.** | **R¹** | **R²** | **L¹** | **L²** | **L³** | **L⁴** | **L⁵** | **X** | **phys. Daten (Fp. [°C])** |
|---|---|---|---|---|---|---|---|---|---|
| I-1 | CH(CH₃)₂ | H | CN | H | H | H | H | Cl | 190 |
| I-2 | -(CH₂)₂CH(CH₃) (CH₂)₂- | | COOCH₃ | F | F | H | H | Cl | 8.7(s); 7.8(d); 3.9(s): 3,7(d); 2,8(t); 1,6(m); 1, 2 (m) ; 0, 9 (d) |
| I-3 | Cyclopentyl | H | CN | H | H | H | H | Cl | 207 |
| I-4 | CH(CH₃)C(CH₃)₃ | H | CN | H | H | H | H | Cl | 130 |
| 1-5 | -(CH₂)₂CH(CH₃) (CH₂)₂- | | CN | F | F | H | H | Cl | 8.4(s); 7.4(d); 3.7(d); 2,8(t); 1,7(m); 1,4(m); 0,9(d) |
| I-6 | CH(CH₃)-CF₃ | H | SCH₃ | F | F | H | H | Cl | 174 |
| I-7 | CH(CH₃)-CF₃ | H | SCH₃ | F | H | F | H | Cl | 164 |
| I-8 | CH₂CF₃ | H | SCH₃ | F | H | F | H | Cl | 161 |

| | | | | | | | | | |
|---|---|---|---|---|---|---|---|---|---|
| Im Fall von chiralen R¹-Gruppen können aufgrund der gehinderten Rotation der Phenylgruppe zwei Diastereomeren A) und B)existieren, die sich in ihren physikalischen Eigenschaften unterscheiden können. | | | | | | | | | |

### Beispiele für die Wirkung gegen Schadpilze

Die fungizide Wirkung der Verbindungen der allgemeinen Formel I ließ sich durch die folgenden Versuche zeigen:

Die Wirkstoffe wurden getrennt oder gemeinsam als 10%ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Wettol® EM (nichtionischer Emulgator auf der Basis von ethoxyliertem Ricinusöl) aufbereitet und entsprechend der gewünschten Konzentration mit Wasser verdünnt.

### Anwendungsbeispiel 1 - Wirksamkeit gegen die Dürrfleckenkrankheit der Tomate, verursacht durch Alternaria solani

Blätter von Topfpflanzen der Sorte "Große Fleischtomate St. Pierre" wurden mit einer wäßrigen Suspension, die aus einer Stammlösung bestehend aus 10 % Wirkstoff, 85 % Cyclohexanon und 5 % Emulgiermittel angesetzt wurde, bis zur Tropfnässe besprüht. Am folgenden Tag wurden die Blätter mit einer wäßrigen Zoosporenaufschwemmung von *Alternaria solani* in 2 % Biomalzlösung mit einer Dichte von 0,17 x 10⁶ Sporen/ml infiziert. Anschließend wurden die Pflanzen in einer Wasserdampf gesättigten Kammer bei Temperaturen zwischen 20 und 22°C aufgestellt. Nach 5 Tagen hatte sich die Krautfäule auf den unbehandelten, jedoch infizierten Kontrollpflanzen so stark entwickelt, daß der Befall visuell in % ermittelt werden konnte.

In diesem Test zeigten die mit 63 ppm des Wirkstoffes I-2 der Tabelle I behandelten Pflanzen keinen Befall, während die unbehandelten Pflanzen zu 90 % befallen waren.

### Anwendungsbeispiel 2 - Wirksamkeit gegen die Netzfleckenkrankheit der Gerste verursacht durch Pyrenophora teres

Blätter von in Töpfen gewachsenen Gerstenkeimlingen der Sorte "Igri" wurden mit wäßriger Wirkstoffaufbereitung, die aus einer Stammlösung bestehend aus 10 % Wirkstoff, 85 % Cyclohexanon und 5 % Emulgiermittel angesetzt wurde, bis zur Tropfnässe besprüht und 24 Stunden nach dem Antrocknen des Spritzbelages mit einer wäßrigen Sporensuspension von *Pyrenophora [syn*. *Drechslera] teres*, dem Erreger der Netzfleckenkrankheit inokuliert. Anschließend wurden die Versuchspflanzen im Gewächshaus bei Temperaturen zwischen 20 und 24°C und 95 bis 100 % relativer Luftfeuchtigkeit aufgestellt. Nach 6 Tagen wurde das Ausmaß der Krankheitsentwicklung visuell in % Befall der gesamten Blattfläche ermittelt.

In diesem Test zeigten die mit 16 ppm des Wirkstoffes 1-2 der Tabelle I behandelten Pflanzen 1 % Befall, während die unbehandelten Pflanzen zu 85 % befallen waren.

## Patentansprüche

1. Triazolopyrimidine der Formel I 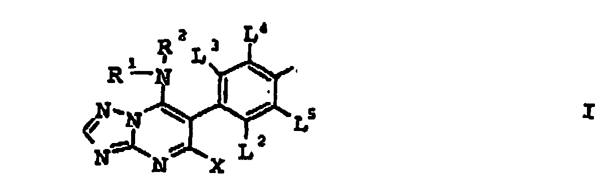 worin die Substituenten folgende Bedeutung haben:
L¹ Cyano oder C(=O)A², worin
A² C₁-C₈-Alkoxy, C₁-C₆-Halogenalkoxy, Wasserstoff, Hydroxy, C₁-C₈-Alkyl, C₁-C₈-Alkylamino oder Di-(C₁-C₈-Alkyl)amino;
L²,L³ Wasserstoff oder Halogen;
L⁴,L⁵ Wasserstoff, Halogen oder C₁-C₄-Alkyl;
X Halogen, Cyano, C₁-C₄-Alkyl, C₁-C₄-Halogenalkyl, C₁-C₄-Alkoxy oder C₁-C₂-Halogenalkoxy;
R¹ C₁-C₆-Alkyl, C₁-C₈-Halogenalkyl, C₃-C₆-Cycloalkyl, C₃-C₆-Halogencycloalkyl, C₂-C₈-Alkenyl, C₂-C₈-Halogenalkenyl, C₂-C₈-Alkinyl; und
R² Wasserstoff oder C₁-C₄-Alkyl; oder
R¹ und R² können auch zusammen mit dem Stickstoffatom, an das sie gebunden sind, einen fünf- oder sechsgliedrigen gesättigten oder ungesättigten Ring bilden, der einen oder zwei Substituenten aus der Gruppe Halogen, C₁-C₆-Alkyl und C₁-C₆-Halogenalkyl tragen kann.

2. Verbindungen der Formel I nach Anspruch 1, in der X Halogen bedeutet.

3. Verfahren zur Herstellung der Verbindungen der Formel gemäß Anspruch 1, in der X für Halogen, Cyano, C₁-C₄-Alkoxy oder C₁-C₂-Halogenalkoxy steht durch Umsetzung von 5-Aminotriazol der Formel II 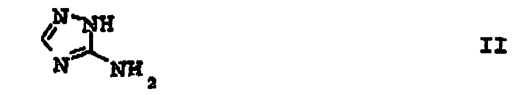 mit Phenylmalonaten der Formel III. 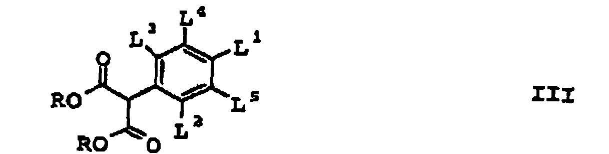 zu Dihydroxytriazolopyrimidinen der Formel IV 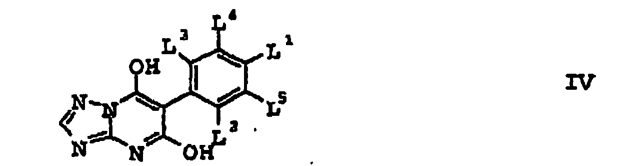 und Halogenierung zu den Dihalogenverbindungen der Formel V, 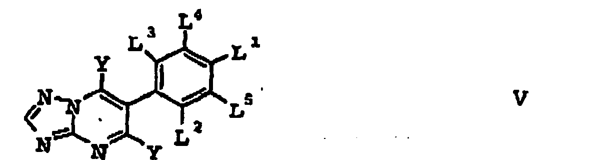 in der Y für Halogen, insbesondere Chlor oder Brom steht, Umsetzung mit Aminen der Formel VI. 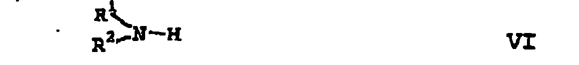 In der R¹ und R² die In Anspruch 1 gegebene Bedeutung haben, zu 5-Halogen-7-aminotriazolopyrimldinen der Formel I, in der X für Halogen steht, und, zur Herstellung von Verbindungen der Formel 1, in der X Cyano, C₁-C₄-Alkoxy oder C₁-C₂-Halegenalkoxy bedeutet, Umsetzung mit Verbindungen der Formel VII,
M-X' VII
die, je nach Bedeutung der einzuführenden Gruppe X', ein anorganisches Cyanid, Alkoxylat oder Halogenalkoxylat darstellt und in der M für ein Ammonium-, Tetraalkylammonium-, Alkalimetall- oder Erdalkalimetallkation steht.

4. Verfahren zur Herstellung der Verbindungen der Formel 1 gemäß Anspruch 1, in der X für C₁-C₄-Alkyl steht, durch Umsetzung von 5-Aminotriazol der Formel II gemäss Anspruch 3 mit Dicarbonylverbindungen der Formel IIIa. 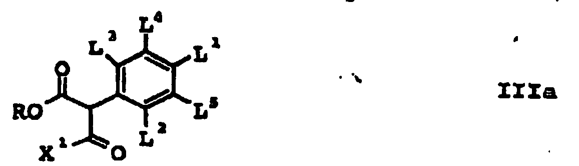 In der R und X¹ für C₁-C₄-Alkyl stehen zu Hydroxytriazolopyrimidinen der Formel IVa 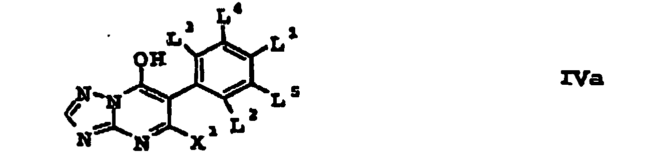 und Halogenierung zu Verbindungen der Formel Va, 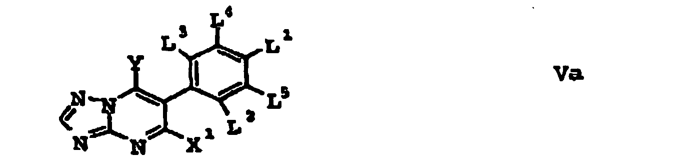 in der Y für Halogen, insbesondere Chlor oder Brom steht, Umsetzung mit Aminen der Formel VI gemäss Anspruch 4 zu Triazolopyrimidinen der Formel 1, in der X für C₁-C₄-Alkyl steht.

5. Verbindungen der Formeln IIIa, IV, IVa, V und Va gemäß den Ansprüchen 3 und 4.

6. Zur Bekämpfung von Schadpilzen geeignetes Mittel, enthaltend einen festen oder flüssigen Trägerstoff und eine Verbindung der Formel 1 gemäß Anspruch 1.

7. Verfahren zur Bekämpfung von Schadpilzen, **dadurch gekennzeichnet dass** man die Pilze oder die vor Pilzbefall zu schützenden Materialien, Pflanzen, den Boden oder Saatgüter mit einer wirksamen Menge einer Verbindung der Formel I gemäß Anspruch 1 behandelt.

## Claims

1. A triazolopyrimidine of the formula I 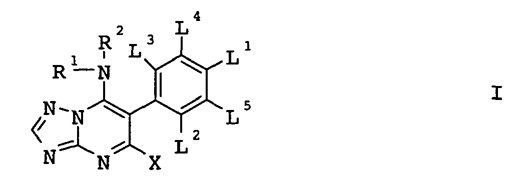 in which the substituents have the following meanings:
L¹ is cyano or C(=O)A², wherein
A² is C₁-C₈-alkoxy, C₁-C₆-haloalkoxy, hydrogen, hydroxyl, C₁-C₈-alkyl, C₁-C₈-alkylamino or di(C₁-C₈-alkyl)amino;
L²,L³ are hydrogen or halogen;
L⁴,L⁵ are hydrogen, halogen or C₁-C₄-alkyl;
X is halogen, cyano, C₁-C₄-alkyl, C₁-C₄-haloalkyl, C₁-C₄-alkoxy or C₁-C₂-haloalkoxy;
R¹ is C₁-C₆-alkyl, C₁-C₈-haloalkyl, C₃-C₆-cycloalkyl, C₃-C₆-halocycloalkyl, C₂-C₈-alkenyl, C₂-C₈-haloalkenyl, C₂-C₈-alkynyl; and
R² is hydrogen or C₁-C₄-alkyl; or
R¹ and R² can also, together with the nitrogen atom to which they are bonded, form a saturated or unsaturated 5- or 6-membered ring which can carry one or two
substituents from the group consisting of halogen, C₁-C₆-alkyl and C₁-C₆-haloalkyl.

2. The compound of the formula I according to claim 1, in which X represents halogen.

3. A process for the preparation of the compounds of the formula I according to claim 1 in which X is halogen, cyano, C₁-C₄-alkoxy or C₁-C₂-haloalkoxy, by reaction of 5-aminotriazole of the formula II 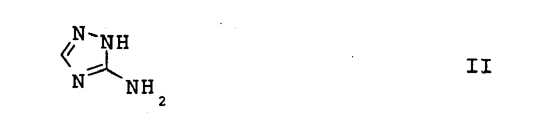 with phenylmalonates of the formula III, 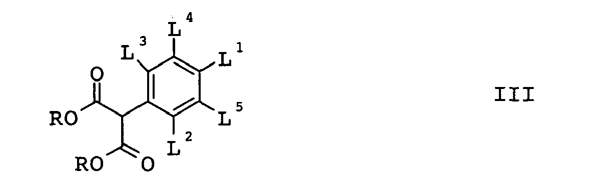 to give dihydroxytriazolopyrimidines of the formula IV 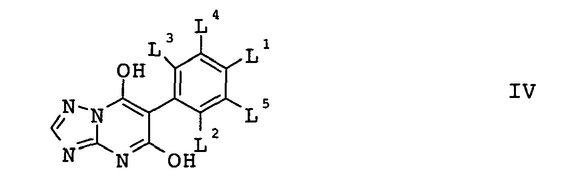 and halogenation to give the dihalogen compounds of the formula V, 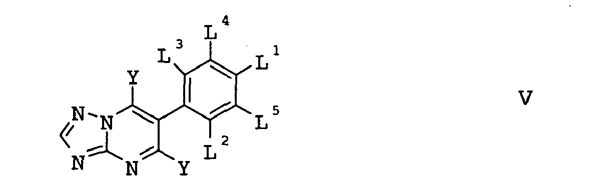 in which Y is halogen, in particular chlorine or bromine, reaction with amines of the formula VI, 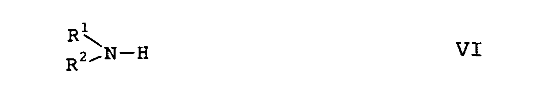 in which R¹ and R² have the meanings given in claim 1, to give 5-halo-7-aminotriazolopyrimidines of the formula I in which X is halogen, and, for the preparation of compounds of the formula I in which X represents cyano, C₁-C₄-alkoxy or C₁-C₂-haloalkoxy, reaction with compounds of the formula VII,
M-X' VII
which, according to the meaning of the group X' to be introduced, represents an inorganic cyanide, alkoxide or haloalkoxide and in which M is an ammonium, tetraalkylammonium, alkali metal or alkaline earth metal cation.

4. A process for the preparation of the compounds of the formula I according to claim 1 in which X is C₁-C₄-alkyl, by reaction of 5-aminotriazole of the formula II according to claim 3 with dicarbonyl compounds of the formula IIIa, 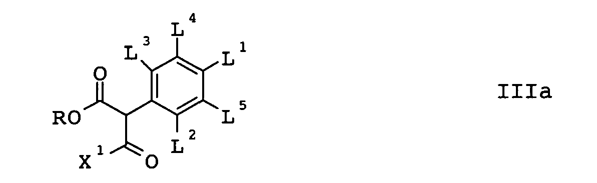 in which R and X¹ are C₁-C₄-alkyl, to give hydroxytriazolopyrimidines of the formula IVa 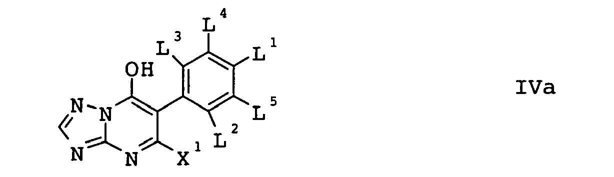 halogenation to give compounds of the formula Va, 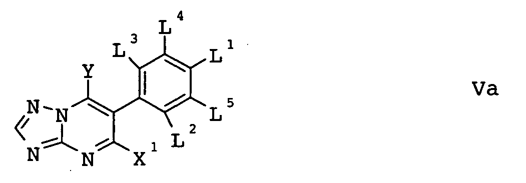 in which Y is halogen, especially chlorine or bromine, and reaction with amines of the formula VI according to claim 4 to give triazolopyrimidines of the formula I in which X is C₁-C₄-alkyl.

5. The compound of the formulae IIIa, IV, IVa, V and Va according to claim 3 or 4.

6. A composition suitable for the control of harmful fungi, comprising a solid or liquid carrier and a compound of the formula I according to claim 1.

7. A method for the control of harmful fungi, which comprises treating the fungi or the materials, plants, ground or seeds to be protected from fungal attack with an effective amount of a compound of the formula I according to claim 1.

## Revendications

1. Triazolopyrimidines de la formule I : 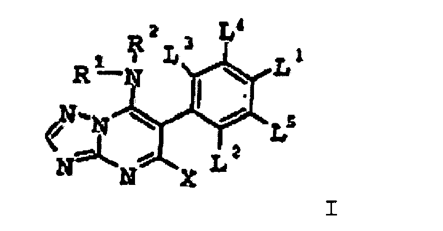 où les substituants ont les significations suivantes :
L¹ cyano ou C(=O)A², où
A² alcoxy en C₁-C₈, halogénoalcoxy en C₁-C₆, hydrogène, hydroxy, alkyle en C₁-C₈(alkyle en C₁-C₈) amino ou di (alkyle en C₁-C₈)amino ;
L², L³ hydrogène ou halogène ;
L⁴, L⁵ hydrogène, halogène ou alkyle en C₁-C₄ ;
X halogène, cyano, alkyle en C₁-C₄, halogénoalkyle en C₁-C₄, alcoxy en C₁-C₄ ou halogénoalcoxy en C₁-C₂ ;
R¹ alkyle en C₁-C₆, halogénoalkyle en C₁-C₈, cycloalkyle en C₃-C₈, halogénocycloalkyle en C₃-C₆, alcényle en C₂-C₈, halogénoalcényle en C₂-C₈, alcynyle en C₂-C₈, et
R² hydrogène ou alkyle en C₁-C₄ ; ou
R¹ et R² peuvent également, avec l'atome d'azote auquel ils sont liés, former un cycle saturé ou insaturé, de 5 ou 6 membres, qui peut porter un ou deux substituants du groupe consistant en halogène, alkyle en C₁-C₆ et halogénoalkyle en C₁-C₆.

2. Composés de la formule I selon la revendication 1, dans lesquels X représente halogène.

3. Procédé de préparation des composés de la formule I selon la revendication 1, dans lequel X représente halogène, cyano, alcoxy en C₁-C₄ ou halogénoalcoxy en C₁-C₂, par réaction du 5-aminotriazole de la formule II : 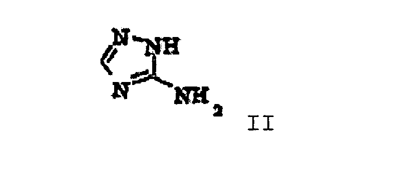 avec des phénylmalonates de la formule III : 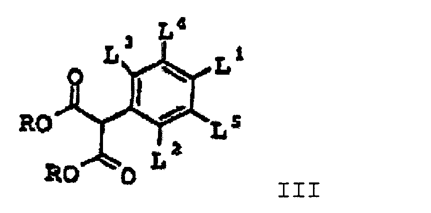 en les dihydroxytriazolopyrimidines de la formule IV : 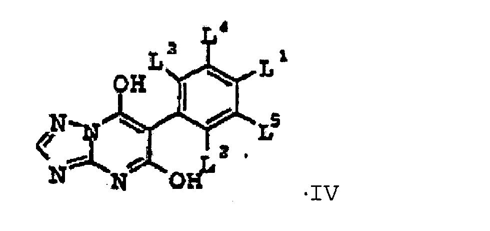 et halogénation en les composés dihalogénés de la formule V : 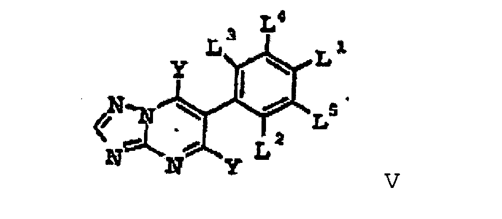 dans laquelle Y représente halogène, en particulier chlore ou brome, réaction avec des amines de la formule VI : 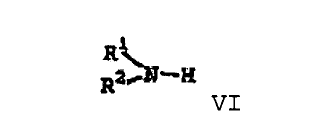 dans laquelle R¹ et R² ont la signification donnée à la revendication 1, en les 5-halogéno-7-aminotriazolopyrimidines de la formule I, dans laquelle X représente halogène, et, pour la préparation de composés de la formule I, dans laquelle X représente cyano, alcoxy en C₁-C₄ ou halogénoalcoxy en C₁-C₂, réaction avec des composés de la formule VII :
M - X' VII
qui, selon la signification du groupe X' introduit, représente un cyanure inorganique, un alcoxylate ou un halogénoalcoxylate et dans laquelle, M représente un cation ammonium, tétraalkylammonium, de métal alcalin ou de métal alcalino-terreux.

4. Procédé de préparation des composés de la formule I selon la revendication 1, dans laquelle X représente alkyle en C₁-C₄, par réaction du 5-aminotriazole de la formule II selon la revendication 3, avec des composés dicarbonylés de la formule IIIa : 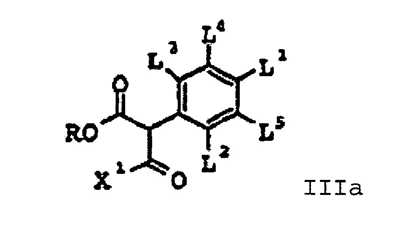 dans laquelle R et X¹ représentent alkyle en C₁-C₄, en les hydroxytriazolopyrimidines de la formule IVa : 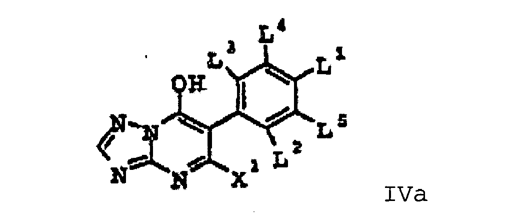 et halogénation en les composés de la formule Va : 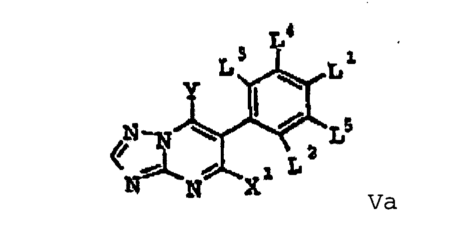 dans laquelle Y représente halogène, et en particulier chlore ou brome, réaction avec les amines de la formule VI selon la revendication 4 en les triazolopyrimidines de la formule I, dans laquelle X représente alkyle en C₁-C₄.

5. Composés des formules IIIa, IV, IVa, V et Va selon les revendications 3 et 4.

6. Agent approprié pour lutter contre les champignons nuisibles, contenant une matière support solide ou liquide et un composé de la formule I selon la revendication 1.

7. Procédé pour lutter contre les champignons nuisibles, **caractérisé en ce que** l'on traite les champignons ou les matériaux, les végétaux, le sol ou les semences, à protéger de l'infestation des champignons, avec une quantité active d'un composé de la formule I selon la revendication 1.
